(19) Europäisches Patentamt
European Patent Office
Office européen des brevets

(11) Publication number: 0 095 916
A2

(12) # EUROPEAN PATENT APPLICATION

(21) Application number: 83303086.9

(22) Date of filing: 27.05.83

(51) Int. Cl.³: A 61 K 7/155

(30) Priority: 01.06.82 US 383431

(43) Date of publication of application: 07.12.83
Bulletin 83/49

(84) Designated Contracting States: BE DE FR GB IT NL

(71) Applicant: THE PROCTER & GAMBLE COMPANY,
301 East Sixth Street, Cincinnati Ohio 45202 (US)

(72) Inventor: Murcia, Daniel Antonio, 3081 Woodside Drive,
Fairfield Ohio 45014 (US)
Inventor: Wells, Robert Lee, 4245 Rose Hill Avenue,
Cincinnati Ohio 45229 (US)

(74) Representative: Brooks, Maxim Courtney et al, Procter & Gamble (NTC) Limited Whitley Road Longbenton,
Newcastle-upon-Tyne NE12 9TS (GB)

(54) **Depilatory compositions.**

(57) A depilatory composition comprised of aminoethanethiol in an aqueous matrix provides fast and effective removal of unwanted body and facial hair. Additionally, a second active thiol agent may be included in the composition.

EP 0 095 916 A2

# DEPILATORY COMPOSITIONS

DANIEL A. MURCIA

ROBERT L. WELLS

## TECHNICAL FIELD

The invention described herein relates to a depilatory composition.

## BACKGROUND

The use of thiol depilatory agents for removal of unwanted body and facial hair is well established in the art. The principle reaction in the chemical removal of hair involves the reduction of hair protein's cystine disulfide groups to sulfhydryl anions. This reaction has been shown to be facilitated by certain conditions such as high alkalinity, resulting in ionized reactants and enhanced penetration by these active agents into the hair.

Although these higher pH's result in faster action, high alkalinity often produces skin irritation for human users. Also, the reaction rate still is not completely acceptable to many consumers. Therefore, faster-acting depilatories which alleviate some of these problems were sought and continue to be sought.

In the search for better-acting depilatories, it was discovered that small thiol molecules bearing low charges provided faster hair removal. It is thought that these small compounds more easily penetrate the hair, thereby reducing time for depilation. The prior art discloses several such compounds, as shown by U.S. Patent 2,990,336, June 27, 1961 to Harry Martin; U.S. Patent 3,865,546, February 11, 1975 to John C. Zemlin and Katherine A. Bowler; U.S. Patent 4,175,922, November 27, 1979 to Guenter Eckert, France Knaflic; Franz-Friedrich Miller and Alfred Zissel; and M. M. Pieger, Depilatories: 1979 update. Cosm. & Toil. 94: 71 - 74 (1979). However, none of this prior art teaches the use of aminoethanethiol, the present depilating agent.

It is an object of the present invention to provide fast and effective removal of unwanted body and facial hair.

It is a further object to provide depilatory compositions containing aminoethanethiol, alone, or in combination with another active thiol agent.

These and other objects will become readily apparent from the detailed description which follows.

All percentages and ratios used herein are by weight, unless otherwise specified.

## SUMMARY OF THE INVENTION

This invention relates to aqueous depilatory compositions comprised of from about 0.1M to about 2.0M aminoethanethiol, alone, or in combination with other active thiol depilatory agents with concentrations ranging from about 0.1M to about 2.0M, such compositions having a pH of from about 10.5 to about 12.5.

## DESCRIPTION OF THE INVENTION

This invention is comprised of two essential ingredients, aminoethanethiol at a level ranging from about 0.10M to about 2.0M, preferably from about 0.2M to about 1.0M, and water. Numerous salts of aminoethanethiol may be used, such as the alkali and alkaline-earth metal salts, the hydrochloride salt, and mixtures thereof, with the hydrochloride being preferred.

As can be seen from the formula below, aminoethanethiol is a small molecule bearing a very low charge. It is felt that these attributes of low molecular weight and minimal charge result in faster and easier penetration by this agent thereby reducing depilation time.

$$H_2N - CH_2 - CH_2 - SH$$

Aminoethanethiol

Water from about 10% to about 80% of the total composition, preferably from about 20% to about 70%, makes up the second essential component of the present invention.

The pH of the aqueous compositions herein should range from about 10.5 to about 12.5 at 25°C., preferably from about 11.0 to about 12.3. The pH's preferably are achieved through the use of an alkaline material such as calcium hydroxide.

Optional components include other active thiol depilatories at concentrations ranging from about 0.10M to about 2.0M, preferably from about 0.20M to about 1.0M. These thiols may include one or more thiol acids, such as thioglycolic, thiolactic acid, and β-mercaptopropionic acid, and/or the alkali or alkaline-earth metal salts of these acids. Other suitable thiol actives which also can be used in the present composition include α-mercaptoethanol, thioglycerols, 1,3-dithio-2-propanol, 1,4-dithio-2-butanol, 1,4-dimercapto-2,3-butanediol, 1,3-dithio-2-methoxypropane, 1,3-dimercapto-2-aminopropane, 1,4-dimercapto-2,3-diaminobutane, related effective thiol actives, and mixtures thereof.

The present invention can take the form of several of the commercial depilatory forms such as creams, lotions, gels, aerosols, and others. As such, additional ingredients relating to such embodiments should be added to the essential component. For instance, suitable fillers, ranging from about 0% to about 5%, can be used. Among suitable fillers are chalk, magnesium oxide and carbonate, clays, such as kaolin, bentonite and related elements, talc, fumed silica, and mixtures thereof.

Emulsifiers which can also be used in the present depilatory composition include anionic surfactants, nonionic surfactants, and mixtures thereof, in ranges from about 0% to about 20%. Examples of the anionic surfactants include fatty alcohol sulfates and alkyl aryl sulfonates, whereas the nonionic types are selected from polyoxyalkylene alcohols and/or ethers. Many additional suitable surfactants are disclosed in McCutheon's <u>Detergents & Emulsifiers</u>, Edition 1979, incorporated herein by reference.

Both natural and synthetic thickeners or gelling agents can be used in the present invention. Such thickeners include tragacanth, xanthan, karaya and guar gums, clays, plus methyl or hydroxyethyl cellulose, hydroxypropyl cellulose, carboxymethyl cellulose, fatty and polyvinyl alcohols, modified starches and sugars, and mixtures thereof. The range of thickener used is from about 0% to about 30%.

Emollients such as paraffin, petrolatum, mineral oil, fatty alcohols, silicone oils, and mixtures thereof are used at levels of from about 0% to about 60%. Fragrances and colorants also are included.

When the present invention is in an emulsion, it may be in either a water-in-oil or oil-in-water form.

Often chelating agents are used to bind unwanted metals in depilatory compositions. Chelating agents suitable for use in the present invention include ethylenediaminotetraacetic acid, its salts and mixtures thereof.

The following examples are presented merely to illustrate the various embodiments of the present invention and are not meant to be a limitation thereof:

## EXAMPLE I

### Depilatory Cream

| | |
|---|---|
| 2-Aminoethanethiol HCl | 7.4800 |
| Calcium Hydroxide | 10.7900 |
| Cabosil HS-5* | 0.5000 |
| Stearyl Alcohol | 7.5000 |
| Isocetyl Alcohol | 5.5000 |
| Brij 56** | 4.3000 |
| Fragrance | 2.0000 |
| Colorant | 0.0125 |
| Water | 61.9175 |

* Fumed Silica

** Polyoxyethylene (10) cetyl ether (ICI Americas, Inc.)

The following example provides an illustration of a depilatory gel containing both aminoethanethiol and another active thiol agent. Again, it merely serves as an example of an embodiment of the present invention and is not limitative of it:

## EXAMPLE II

### Depilatory Gel

| | |
|---|---|
| Ceteareth 50* | 24.50 |
| Thioglycerol | 2.48 |
| 2-aminoethanethiol HCl | 5.00 |
| Calcium Hydroxide | 10.80 |
| Fragrance | 2.00 |
| Water | 55.22 |

*Polyethylene glycol ether of cetearyl alcohol

Another embodiment of the present invention is as an aerosol. Along with the previously-listed ingredients of the various depilatory embodiments, aerosols utilize propellants. The following example presents an example of an aerosol:

## EXAMPLE III

### Depilatory Aerosol

| A. Concentrate | Wt. % |
|---|---|
| Calcium Thioglycolate | 3.00 |
| Aminoethanethiol | 4.75 |
| Calcium Hydroxide | 10.80 |
| Polyoxyethylene(20)Stearyl Ether | 4.30 |
| Stearyl Alcohol | 7.50 |
| Mineral Oil | 3.00 |
| Vaseline | 3.00 |
| Fragrance | 0.30 |
| Water | 63.35 |

EXAMPLE III (continued)

| B. Fill | Grams |
|---|---|
| Concentrate | 175.00 |
| Propellant 12 | 7.12 |
| Propellant 114 | 5.38 |

Claims

1. An aqueous depilatory composition characterised by from 0.1M to 2.0M of aminoethanethiol or its salts, wherein said composition has a pH of from 10.5 to 12.5.

2. A depilatory composition according to Claim 1 characterised by from 0.2M to 1.0M aminoethanethiol or its salts.

3. A depilatory composition according to Claim 1 or 2 characterised in that the composition has a pH of from 11.0 to 12.3.

4. A depilatory composition according to any of Claims 1 to 3 characterised in that the composition additionally contains from 0% to 5% of a filler, from 0% to 20% of an emulsifier, from 0% to 30% of a gelling agent or thickener, from 0% to 60% of an emollient, from 0% to 5% of a chelating agent, with fragrance, colorant, and water making up the remainder.

5. A depilatory composition according to Claim 4 characterised in that the filler is selected from chalk, magnesium oxide and carbonate, clays, talc, fumed silica, and mixtures thereof, the emulsifier is selected from anionic surfactants, nonionic surfactants, and mixtures thereof, the thickener is selected from tragacanth, xanthan, karaya, and guar gums, clays, methyl or hydroxyethyl cellulose, hydroxypropyl cellulose, carboxymethyl cellulose, fatty and polyvinyl alcohols, modified starches and sugars, and mixtures thereof, the emollient is selected from paraffin, petrolatum, mineral oil, fatty alcohols, silicone oils and mixtures thereof, and the chelating agent is selected from ethylenediaminetetraacetic acid, the salts of said acid, and mixtures thereof.

0095916

6. A depilatory composition according to any of Claims 1 to 5 characterised in that the composition additionally contains from 0.1M to 2.0M of a second active thiol depilatory.

7. A depilatory composition according to Claim 6 characterised in that the second active thiol agent is present at a level of from 0.2M to 1.0M.

8. A depilatory composition according to Claim 7 characterised in that the second active thiol agent is selected from thioglycolic acid, thiolactic acid, β-mercaptopropionic acid, the alkali or alkaline-earth metal salts of these acids, α-mercaptoethanol, thioglycerol, 1,3-dithio-2-propanol, 1,4-dithio-2-butanol, 1,4-dimercapto-2,3-butanediol, 1,3-dithio-2-methoxypropane, 1,3-dimercapto-2-aminopropane, 1,4-dimercapto-2,3-diaminobutane, and mixtures thereof.

9. A depilatory composition according to any preceding claim in the form of an aerosol wherein said composition additionally contains from 0% to 20% of a propellant.